# EUROPEAN PATENT APPLICATION

(11) **EP 3 546 973 A1**
(43) Date of publication of application: **02.10.2019**
(21) Application number: 18163886.7
(22) Date of filing: 26.03.2018
(51) Int. Cl.: G01R 33/54, A61B 5/00, G01R 33/565, G05B 13/02, A61B 5/055

(54) **PREDICTIVE MODIFICATION OF MAGNETIC RESONANCE IMAGING PROTOCOLS**

(71) Applicant: Koninklijke Philips N.V., 5656 AE Eindhoven (NL)
(72) Inventor: AMTHOR, Thomas Erik, 5656 AE Eindhoven (NL); BORGERT, Jörn, 5656 AE Eindhoven (NL); HELLE, Michael Günter, 5656 AE Eindhoven (NL); NETSCH, Thomas, 5656 AE Eindhoven (NL); HANSIS, Eberhard Sebastian, 5656 AE Eindhoven (NL)
(74) Representative: de Haan, Poul Erik

(57) **Abstract**

The invention provides for a magnetic resonance imaging system (100) that comprises a sensor system (122) for measuring subject data (156) and a user interface (132). Machine executable instructions (140) causes a processor (134) to receive (200) a set of examination commands (142) and initiate (202) execution of them. Before executing each magnetic resonance imaging protocol of the set of examination commands, the processor repeatedly: receives (204) the subject data; determines (206) an examination status (154) using the set of examination commands; calculates (208) at least one scan decision probability (160) using a machine learning algorithm (158) by inputting the subject data and the examination status; pauses (212) execution of the set of examination commands if the at least one scan decision probability is within one or more predetermined probability ranges (162); displays (214) a user prompt (164, 164', 164") on the user interface if the at least one scan decision probability is within the one or more predetermined probability ranges; receives (216) the modification instructions from the user interface; modifies (218) the set of examination commands; and resumes (219) execution of the set of examination commands.

## Description

### FIELD OF THE INVENTION

The invention relates to magnetic resonance imaging, in particular to the avoidance of image degradation in magnetic resonance imaging.

### BACKGROUND OF THE INVENTION

A large static magnetic field is used by Magnetic Resonance Imaging (MRI) scanners to align the nuclear spins of atoms as part of the procedure for producing images within the body of a patient. This large static magnetic field is referred to as the B0 field or the main magnetic field. Various quantities or properties of the subject can be measured spatially using MRI. For example, various electrical properties of a subject can be investigated using MRI. However, the acquisition of magnetic resonance data for imaging is not instantaneous. For example, the motion of a subject either voluntarily or involuntarily can result in artifacts or blurring in magnetic resonance images. Often time a subject will be imaged multiple times during a magnetic resonance imaging session using several different imaging protocols. As more imaging protocols are performed it can be challenging to acquire magnetic resonance imaging that are free of artifacts due to subject motion.

United States patent application publication US 2015/0212182 A1 discloses a magnetic resonance imaging system for acquiring magnetic resonance data. A processor for controlling the magnetic resonance imaging system executes instructions which cause the processor to repeatedly: control the magnetic resonance imaging system to acquire a portion of the magnetic resonance data, wherein each portion of the magnetic resonance data comprises navigator data; create a set of navigator vectors by extracting the navigator data from each portion of the magnetic resonance data; construct a dissimilarity matrix by calculating a metric between each of the set of navigator vectors; generate a matrix classification of the dissimilarity matrix using a classification algorithm; and control the magnetic resonance imaging system to modify acquisition of the magnetic resonance data using the matrix classification.

### SUMMARY OF THE INVENTION

The invention provides for a magnetic resonance imaging system, a computer program product, and a method in the independent claims. Embodiments are given in the dependent claims.

Often times, when a subject undergoes a magnetic resonance imaging (MRI) examination multiple imaging protocols are employed to acquire magnetic resonance data to reconstruct magnetic resonance images. A difficulty is that sometimes one or more of these protocols must be aborted due to the subject or the subject moves part way through a protocol and spoils the acquisition of the magnetic resonance imaging data. Embodiments may provide for a means of reducing the chance that magnetic resonance imaging protocols are aborted and/or acquired magnetic resonance images contain artifacts or are degraded due to motion of the subject. This may be accomplished by first having a sensor system that acquires subject data descriptive of a state of the subject and determining an examination status from a set of examination commands. The set of examination commands comprise the various pulse sequence instructions that are used to implement the acquisition of magnetic resonance imaging data for each of the magnetic resonance imaging protocols. The examination status may include information or details about the present state of the MRI examination such as protocols to be performed, protocols that have already been performed, and the duration the subject has already been in the magnetic resonance imaging system. The subject data may include data which could be used to measure the current physiological state of the subject, psychological state of the subject, position of the subject, and/or movement of the subject.

The subject data and the examination status are then input into a machine learning algorithm which outputs one or more scan decision probabilities before each magnetic resonance imaging protocol is initiated by executing its pulse sequence commands. The one or more scan decision probabilities are probabilities that predict various events that could for example cause an abort or degradation of the magnetic resonance imaging data. For example the subject chooses to abort the protocol or the subject changes position spoiling the acquisition of the magnetic resonance imaging data. If the one or more scan decision probabilities are within one or more predetermined probability ranges, it triggers a pause of the set of examination commands before the next magnetic resonance imaging protocol is performed (before its pulse sequence instructions are executed). A user prompt is displayed on a user interface and suggests modifications to the set of examination commands or suggests an action by the operator to reduce that chances that the next protocol is aborted or spoiled by movement.

In one aspect the invention provides for a magnetic resonance imaging system configured for imaging a subject. The magnetic resonance imaging system further comprises a sensor system for measuring subject data descriptive of a subject condition. The subject condition may for example be descriptive of subject motion that is either voluntary or involuntary. The subject condition may also include other parameters which are descriptive of a state or condition of the subject such as behavior or previous motion of the subject. In one example the subject condition is descriptive of motion of the subject. The magnetic resonance imaging system further comprises a user interface configured for displaying messages and receiving modification instructions. In one example the user interface is a graphical user interface that may be adapted for displaying messages and receiving the control commands.

The magnetic resonance imaging system further comprises a memory for storing machine-executable instructions. The magnetic resonance imaging system further comprises a processor configured for controlling the magnetic resonance imaging system. Execution of the machine-executable instructions causes the processor to receive a set of examination commands. The set of examination commands comprise pulse sequence instructions for controlling the magnetic resonance imaging system to image a subject according to one or more magnetic resonance imaging protocols. For example, when a subject is placed into a magnetic resonance imaging system normally a collection or set of examination commands are performed. Each of the set of examination commands may for example be the execution of one magnetic resonance imaging protocol. For each magnetic resonance imaging protocol that is performed there may be separate pulse sequence instructions or modifications of different parameters of the pulse sequence instructions. Execution of the machine-executable instructions further cause the processor to initiate execution of the set of examination commands.

Execution of the machine-executable instructions further cause the processor to repeatedly perform the following before executing the pulse sequence instructions of each of the one or more magnetic resonance imaging protocols. That is to say the following steps are performed before each of the magnetic resonance imaging protocols is started. First execution of the machine-executable instructions causes the processor to receive the subject data from the sensor system. Execution of the machine-executable instructions further cause the processor to determine an examination status using the set of examination commands. The examination status may for example be descriptive of the present state or process in the execution of the set of examination commands. The examination status may for example indicate which magnetic resonance imaging protocols have been performed and which ones have not been performed.

The examination status may also contain other information such as the total lapsed time since the subject has been placed into the magnetic resonance imaging system. Execution of the machine-executable instructions further cause the processor to calculate at least one scan decision probability using a machine learning algorithm. Input to the machine learning algorithm comprises the subject data and the examination status. The at least one scan decision probability is output of the machine learning algorithm. Execution of the machine-executable instructions further cause the processor to pause execution of the set of examination commands if the at least one scan decision probability is within one or more predetermined probability ranges. For each of the at least one scan decision probability there is a predetermined probability range.

Execution of the machine-executable instructions further cause the processor to display a user prompt on the user interface if the at least one scan decision probability is within the one or more predetermined probability ranges. Execution of the machine-executable instructions further cause the processor to receive modification instructions from the user interface if the user prompt is displayed. Execution of the machine-executable instructions further cause the processor to modify the set of examination commands if the modification instructions are received. Execution of the machine-executable instructions further cause the processor to resume execution of the set of examination commands if the modification instructions have been modified using the modification instructions.

This embodiment may be beneficial because it may provide a means to correct or modify a magnetic resonance imaging protocol before it is performed. The machine learning algorithm uses the subject data and the examination status as input and then outputs the at least one scan decision probability. The at least one scan decision probability may indicate various things such as a fault or predict likely movement of the subject which may cause problems in the acquisition of magnetic resonance imaging data.

If the at least one scan decision probability is within the one or more predetermined probability ranges then it triggers the display of the user interface and the receiving of data from the user interface that is used to modify the set of examination commands. This may have the benefit of reducing the failures or aborts of the acquisition of magnetic resonance data. This check of the at least one scan decision probability is performed before each of the one or more magnetic resonance imaging protocols are executed. The machine learning algorithm is therefore used to predict problems which may affect the acquisition of the magnetic resonance imaging data before it occurs. This may allow modification of the set of examination commands to reduce the chance of failures or aborts.

In another embodiment the magnetic resonance imaging system further comprises a historical database comprising entries. Each of the entries is descriptive of a prior set of examination commands correlated with historical subject data. Each of the entries further comprises a scan status indicating a success, an abort or repeat of the prior set of examination commands. This embodiment may be beneficial because it may provide for data which may be used by the machine learning algorithm.

In another embodiment execution of the machine-executable instructions further cause the processor to train the machine learning algorithm using the historical database. The machine learning algorithm is in one example a statistical classification algorithm. In another example the machine learning algorithm is a neural network. In another example the machine learning algorithm is a pattern recognition algorithm. In another embodiment the machine learning algorithm is a deep learning algorithm. In another example the machine learning algorithm is a clustering algorithm. In another example the machine learning algorithm is a k nearest neighbors' algorithm. In another example the machine learning algorithm is a Bayesian network.

In another embodiment execution of the machine-executable instructions further cause the processor to generate a log entry for the set of examination commands, the user data, and the scan status of the set of examination commands after execution of the set of examination commands is finished. Execution of the machine-executable instructions further causes the processor to perform a feature extraction on the log entry. Execution of the machine-executable instructions further causes the processor to append a feature extraction on the log entry to the historical database as an additional entry. This embodiment may be beneficial because the historical database is continually added to as the magnetic resonance imaging system is used and this is used to further train the machine learning algorithm. As the magnetic resonance imaging system is used the predictive power of the machine learning algorithm is continually improved.

In another embodiment the user prompt displays a decrease resolution message if the at least one scan decision probability is within a first range. The modification instruction causes the set of examination commands to decrease an imaging resolution of the pulse sequence instructions. This for example may be useful in reducing the amount of time to acquire the magnetic resonance data for a particular magnetic resonance imaging protocol. If for example the at least one scan decision probability predicts that the subject will move during the acquisition of magnetic resonance data the chance that the magnetic resonance data can be acquired successfully can be increased by decreasing the resolution.

In another embodiment the user prompt displays an increase resolution message if the at least one scan decision probability is below a predetermined lower value. The modification instructions cause the set of examination commands to increase the imaging resolution of the pulse sequence instructions. This for example may be useful in a situation where the at least one scan decision probability indicates that the probability that a subject will move during the acquisition of magnetic resonance data is relatively small. In this case it may be reasonable to assume that the duration of a particular magnetic resonance imaging protocol can be increased. This may be useful because then the resolution can be increased which may provide for better quality magnetic resonance images.

In another embodiment the prompt comprises a selection of an alternative magnetic resonance imaging protocol. This for example may be useful when the at least one scan decision probability predicts that a particular magnetic resonance imaging protocol will not be completed or will be aborted. The selection of alternative magnetic resonance imaging protocol might be useful therefore for selecting a protocol which requires less time.

In another embodiment the prompt displays a choice or suggestion to terminate execution of the set of examination commands.

In another embodiment the user prompt displays a choice or a suggestion to change an ordering of remaining magnetic resonance imaging protocols. This for example may be useful when changing the ordering results in a reduced likelihood that there will be aborts of particular magnetic resonance imaging protocols.

In another embodiment the user prompt displays a choice or suggestion to delay execution of the set of examination commands until the subject relaxes. For example, if the subject has elevated heart rate or other excessive movement then this may indicate that the subject may not be able to complete a magnetic resonance imaging protocol. By delaying execution, it may be possible for the subject to relax and increase the probability that a magnetic resonance imaging protocol is completed successfully.

In another embodiment the user prompt displays a choice or suggestion to skip execution of one or more magnetic resonance imaging protocols. For example, if it is predicted that it is not likely that a magnetic resonance imaging protocol will be completed, it is possible to skip the execution of this and avoid wasting time on an aborted magnetic resonance imaging acquisition.

In another embodiment the user prompt displays a choice or suggestion to speak with the subject. Surprisingly, in many cases, the prediction of whether a subject will complete a magnetic resonance imaging protocol successfully or not maybe related to how much the operator of the magnetic resonance imaging system has spoken with the subject. This for example may have a calming effect on the subject. If the protocol is likely not to be completed, it may be possible to increase the probability of completion if the operator speaks with the subject.

In another embodiment the examination status comprises a list of remaining magnetic resonance imaging protocols to be performed.

In another embodiment the examination status comprises magnetic resonance imaging protocols that have already been performed. For example, the remaining protocols and the protocols already performed may be useful in estimating a probability that the subject will not be able to complete the examination.

In another embodiment the examination status comprises a time elapsed since initiation of the set of examination commands. A predictor of when the examination will fail may be how long the subject has been within the magnetic resonance imaging system in total. For example, the longer a child or an elderly person is in a magnetic resonance imaging system it may become more likely that they are uneasy and have more stress. The time elapsed since initiation of the set of examination commands or when the procedure started may be a useful measure of predicting if a subject will move and ruin a magnetic resonance imaging acquisition.

In another embodiment the examination status comprises a type of next magnetic resonance imaging protocol to be performed. For example, particular magnetic resonance imaging protocols may be particularly loud or startling for some subjects. The type of the magnetic resonance imaging protocol may therefore have an effect on how well the subject is able to complete the magnetic resonance imaging protocol.

In another embodiment the subject data comprises any subject-specific data accessible through a hospital network. This for example may comprise a medical history or data descriptive of the subject.

In another embodiment the subject data comprises an age of the subject. For example, younger subjects may be less likely to be able to remain still during the duration of many magnetic resonance imaging protocols.

In another embodiment the subject data comprises a weight of the subject. For example, the weight of the subject may have an impact on how comfortable the subject is within a magnetic resonance imaging system.

In another embodiment the subject data further comprises a height of the subject. Again, the height may affect how comfortable the subject is within the magnetic resonance imaging system.

In another embodiment the subject data further comprises a health status of the subject. For example, if the subject is suffering from a chronic disease or has another condition which may affect the subject's comfort within the magnetic resonance imaging system, this may have an effect on how well the subject is able to complete a magnetic resonance imaging protocol.

In another embodiment the subject data further comprises a medical history of the subject. In this embodiment the medical history and other data of the subject may affect how well they are able to stay still during the execution of a magnetic resonance imaging protocol.

In another embodiment the subject data further comprises responses from one or more questionnaire responses from the subject. For example, before the subject is placed into the magnetic resonance imaging system the subject maybe asked a number of questions on a questionnaire. These questions may be used to estimate the comfort level of the subject within the magnetic resonance imaging system and may therefore have an effect on whether the subject is able to complete a magnetic resonance imaging protocol or not.

In another embodiment execution of the machine-executable instructions further cause the processor to calculate the subject data at least partially from the magnetic resonance data acquired during execution of the set of examination commands. For example, once one or more magnetic resonance imaging protocols have been performed there is magnetic resonance data which can be examined. This magnetic resonance data for example may be used to give a quantitative measure of how much the subject has moved during prior magnetic resonance examinations. This may be a useful predictor of future behavior of the subject.

In another embodiment the sensor system comprises an optical finger pulse sensor configured for monitoring a subject heart rate. This embodiment may be beneficial because the heart rate of the subject or even a change in the heart rate may be a predictor if the subject is able to complete a magnetic resonance imaging protocol.

In another embodiment the sensor system comprises an ECG system configured for monitoring the subject heart rate. Likewise, the ECG system may be used to predict changes in the subject's comfort level or the current state of the subject.

In another embodiment the sensor system comprises a respiratory belt configured for monitoring a subject breathing state. For example, the rate of breathing of the subject or a change in the rate of breathing of the subject maybe useful in predicting if a subject can complete a magnetic resonance imaging protocol.

In another embodiment the sensor system comprises a breathing tube configured for monitoring the subject's breathing state. The monitoring of the subject breathing rate or a change in the subject breathing rate again may be useful in estimating if a subject can complete a magnetic resonance imaging protocol.

In another embodiment the sensor system comprises a heart rate camera configured for measuring the subject heart rate using facial color changes. This embodiment may be beneficial because there is no contact with the subject and the heart rate camera system may be useful in estimating if a subject is able to complete a magnetic resonance imaging protocol.

In another embodiment the sensor system comprises a motion monitor camera system configured for measuring a subject position. Changes in the subject position may be useful in estimating if a subject can remain still enough during the acquisition of magnetic resonance data. The motion monitor camera may take different forms in different examples. For example, the motion monitor camera may be a simple black and white or color camera which monitors the change in pixels. In other examples the motion monitor camera may be a 3D camera and used for monitoring the motion of a subject.

In another embodiment the sensor system comprises a breathing monitor camera system configured for measuring the subject breathing state. For example, the breathing monitor camera may take an image of the subject's chest or even a receive coil that has been placed on the chest of the subject. The breathing monitor camera maybe able to monitor small changes in the subject's breathing state or rate without contact with the subject.

In another embodiment the sensor system comprises a skin resistance system configured for measuring skin resistance data of the subject. Changes or the values of the skin resistance data may be useful in predicting how comfortable a subject will be during a future acquisition of magnetic resonance data during one of the magnetic resonance imaging protocols.

In another embodiment the sensor system comprises a microphone system configured for quantifying a cumulative speaking duration of the subject. For example, the microphone system may be attached to a speech recognition system that is able to measure how much talking has occurred between a subject and the operator of the magnetic resonance imaging system. This could be further used to quantify how much speaking the operator has made and how much speaking in response the subject has made. For example, if the operator is speaking to the subject but the subject is not answering very much, this can be quantitatively measured and input into the machine learning algorithm.

In another aspect the invention provides for a computer program product comprising machine-executable instructions for execution by a processor controlling the magnetic resonance imaging system. The magnetic resonance imaging system comprises a sensor system for measuring subject data descriptive of a subject condition. The magnetic resonance imaging system further comprises a user interface configured for displaying messages and receiving modification instructions. Execution of the machine-executable instructions causes the processor to receive a set of examination commands. The set of examination commands comprise pulse sequence instructions for controlling the magnetic resonance imaging system to image the subject according to one or more magnetic resonance imaging protocols. Execution of the machine-executable instructions further cause the processor to initiate execution of the set of examination commands.

Execution of the machine-executable instructions further causes the processor to repeatedly perform the following steps before executing the pulse sequence instructions of each of the one or more magnetic resonance imaging protocols. The instructions cause the processor to receive the subject's data from the sensor system. Execution of the machine-executable instructions further cause the processor to determine an examination status using the set of examination commands. Execution of the machine-executable instructions further cause the processor to calculate at least one scan decision probability using a machine learning algorithm.

Input to the machine learning algorithm comprises the subject data and the examination status. The at least one decision scan probability is the output of the machine learning algorithm. Execution of the machine-executable instructions further cause the processor to pause execution of the set of examination commands if the at least one scan decision probability is within one or more predetermined probability ranges. Execution of the machine-executable instructions further cause the processor to display a user prompt on the user interface if the at least one scan decision probability is within the one or more predetermined probability ranges. Execution of the machine-executable instructions further cause the processor to receive modification instructions from the user interface if the user prompt is displayed.

Execution of the machine-executable instructions further cause the processor to modify the set of examination commands if modification instructions are received.

Execution of the machine-executable instructions further cause the processor to resume execution of the set of examination commands if the modification instructions have been modified using the modification instructions.

In another aspect the invention provides for a method of operating a magnetic resonance imaging system. The magnetic resonance imaging system comprises a sensor system for measuring subject data descriptive of a subject condition. The magnetic resonance imaging system further comprises a user interface configured for displaying messages and receiving modification instructions. The method comprises receiving a set of examination commands. The set of examination commands comprises pulse sequence instructions for controlling the magnetic resonance imaging system to image the subject according to one or more magnetic resonance imaging protocols. The method further comprises initiating execution of the set of examination commands.

The method further comprises repeatedly performing the following steps before executing the pulse sequence instructions of each of the one or more magnetic resonance imaging protocols. The method comprises receiving the subject data from the sensor system. The method further comprises determining an examination status using the set of examination commands. The method further comprises calculating at least one scan decision probability using a machine learning algorithm. The input to the machine learning algorithm comprises the subject data and the examination status. The at least one scan decision probability is output of the machine learning algorithm.

The method further comprises pausing execution of the set of examination commands if the at least one scan decision probability is within one or more predetermined probability ranges. The method further comprises displaying a user prompt on the user interface if the at least one scan decision probability is within one or more predetermined probability ranges. The method further comprises receiving modification instructions from the user interface if the user prompt is displayed. The method further comprises modifying the set of examination commands if the modification instructions are received. The method further comprises resuming execution of the set of examination commands if the modification instructions have been modified using the modification instructions.

It is understood that one or more of the aforementioned embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, microcode, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) maybe utilized. The computer readable medium may be a computer readable signal medium or a computer readable storage medium. A 'computer-readable storage medium' as used herein encompasses any tangible storage medium which may store instructions which are executable by a processor of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the processor of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the processor. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example a data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

'Computer memory' or 'memory' is an example of a computer-readable storage medium. Computer memory is any memory which is directly accessible to a processor. 'Computer storage' or 'storage' is a further example of a computer-readable storage medium. Computer storage is any non-volatile computer-readable storage medium. In some embodiments computer storage may also be computer memory or vice versa.

A 'processor' as used herein encompasses an electronic component which is able to execute a program or machine executable instruction or computer executable code. References to the computing device comprising "a processor" should be interpreted as possibly containing more than one processor or processing core. The processor may for instance be a multi-core processor. A processor may also refer to a collection of processors within a single computer system or distributed amongst multiple computer systems. The term computing device should also be interpreted to possibly refer to a collection or network of computing devices each comprising a processor or processors. The computer executable code may be executed by multiple processors that may be within the same computing device or which may even be distributed across multiple computing devices.

Computer executable code may comprise machine executable instructions or a program which causes a processor to perform an aspect of the present invention. Computer executable code for carrying out operations for aspects of the present invention may be written in any combination of one or more programming languages, including an object oriented programming language such as Java, Smalltalk, C++ or the like and conventional procedural programming languages, such as the "C" programming language or similar programming languages and compiled into machine executable instructions. In some instances the computer executable code may be in the form of a high level language or in a pre-compiled form and be used in conjunction with an interpreter which generates the machine executable instructions on the fly.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection maybe made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further under stood that, when not mutually exclusive, combinations of blocks in different flowcharts, illustrations, and/or block diagrams may be combined. These computer program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks.

These computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A 'user interface' as used herein is an interface which allows a user or operator to interact with a computer or computer system. A 'user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A 'hardware interface' as used herein encompasses an interface which enables the processor of a computer system to interact with and/or control an external computing device and/or apparatus. A hardware interface may allow a processor to send control signals or instructions to an external computing device and/or apparatus. A hardware interface may also enable a processor to exchange data with an external computing device and/or apparatus. Examples of a hardware interface include, but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen, Cathode ray tube (CRT), Storage tube, Bi-stable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

Magnetic Resonance (MR) data is defined herein as being the recorded measurements of radio frequency signals emitted by atomic spins using the antenna of a Magnetic resonance apparatus during a magnetic resonance imaging scan. MRF magnetic resonance data is magnetic resonance data. Magnetic resonance data is an example of medical image data. A Magnetic Resonance Imaging (MRI) image or MR image is defined herein as being the reconstructed two or three-dimensional visualization of anatomic data contained within the magnetic resonance imaging data. This visualization can be performed using a computer.

### BRIEF DESCRIPTION OF THE DRAWINGS

In the following preferred embodiments of the invention will be described, by way of example only, and with reference to the drawings in which:
- Fig. 1: illustrates an example of a magnetic resonance imaging system;
- Fig. 2: shows a flow chart which illustrates a method of operating the magnetic resonance imaging system of Fig. 1;
- Fig. 3: illustrates an example of a user prompt;
- Fig. 4: illustrates a further example of a user prompt;
- Fig. 5: illustrates the collection of training information; and
- Fig. 6: illustrates a real-time prediction method.

### DETAILED DESCRIPTION OF THE EMBODIMENTS

Like numbered elements in these figures are either equivalent elements or perform the same function. Elements which have been discussed previously will not necessarily be discussed in later figures if the function is equivalent.

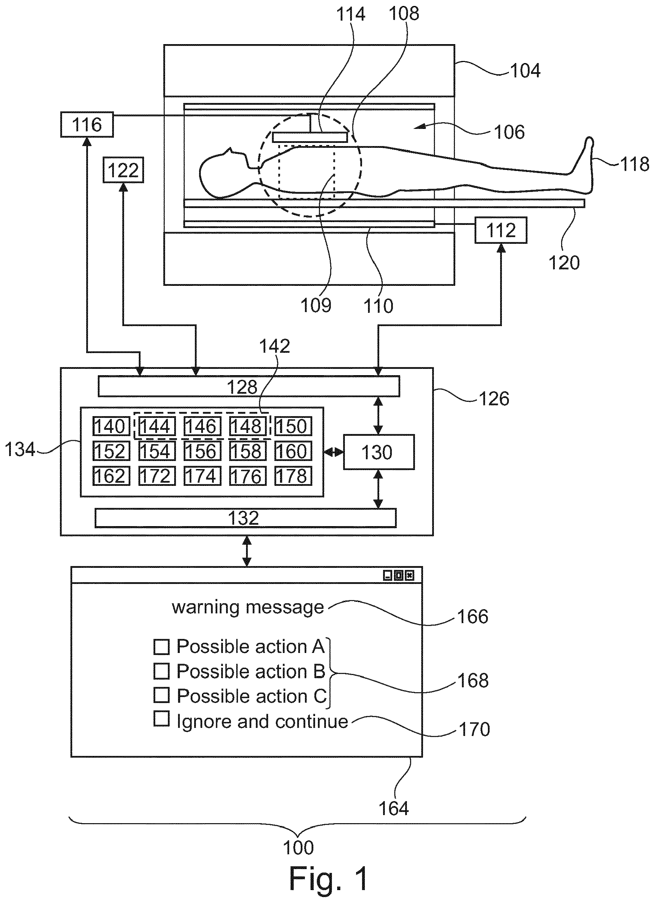

Fig. 1 shows an example of a magnetic resonance imaging system 100 with a magnet 104. The magnet 104 is a superconducting cylindrical type magnet with a bore 106 through it. The use of different types of magnets is also possible; for instance it is also possible to use both a split cylindrical magnet and a so called open magnet. A split cylindrical magnet is similar to a standard cylindrical magnet, except that the cryostat has been split into two sections to allow access to the iso-plane of the magnet, such magnets may for instance be used in conjunction with charged particle beam therapy. An open magnet has two magnet sections, one above the other with a space in-between that is large enough to receive a subject: the arrangement of the two sections area similar to that of a Helmholtz coil. Open magnets are popular, because the subject is less confined. Inside the cryostat of the cylindrical magnet there is a collection of superconducting coils. Within the bore 106 of the cylindrical magnet 104 there is an imaging zone 108 where the magnetic field is strong and uniform enough to perform magnetic resonance imaging. A region of interest 109 is shown within the imaging zone 108. The magnetic resonance data that is acquired typically acquried for the region of interest. A subject 118 is shown as being supported by a subject support 120 such that at least a portion of the subject 118 is within the imaging zone 108 and the region of interest 109.

Within the bore 106 of the magnet there is also a set of magnetic field gradient coils 110 which is used for acquisition of preliminary magnetic resonance data to spatially encode magnetic spins within the imaging zone 108 of the magnet 104. The magnetic field gradient coils 110 connected to a magnetic field gradient coil power supply 112. The magnetic field gradient coils 110 are intended to be representative. Typically magnetic field gradient coils 110 contain three separate sets of coils for spatially encoding in three orthogonal spatial directions. A magnetic field gradient power supply supplies current to the magnetic field gradient coils. The current supplied to the magnetic field gradient coils 110 is controlled as a function of time and may be ramped or pulsed.

Adjacent to the imaging zone 108 is a radio-frequency coil 114 for manipulating the orientations of magnetic spins within the imaging zone 108 and for receiving radio transmissions from spins also within the imaging zone 108. The radio frequency antenna may contain multiple coil elements. The radio frequency antenna may also be referred to as a channel or antenna. The radio-frequency coil 114 is connected to a radio frequency transceiver 116. The radio-frequency coil 114 and radio frequency transceiver 116 may be replaced by separate transmit and receive coils and a separate transmitter and receiver. It is understood that the radio-frequency coil 114 and the radio frequency transceiver 116 are representative. The radio-frequency coil 114 is intended to also represent a dedicated transmit antenna and a dedicated receive antenna. Likewise the transceiver 116 may also represent a separate transmitter and receivers. The radio-frequency coil 114 may also have multiple receive/transmit elements and the radio frequency transceiver 116 may have multiple receive/transmit channels. For example if a parallel imaging technique such as SENSE is performed, the radio-frequency could 114 will have multiple coil elements.

Fig. 1 shows a sensor system 122. 122 is intended to represent one or more different sensor systems. For example, 122 may represent an optical finger pulse sensor, an ECG system, a respiratory belt, a breathing tube, a heart rate camera system, a motion monitor camera system, a breathing monitor camera system, a skin resistance system, and/or a microphone system.

The transceiver 116, the gradient controller 112, and the sensor system 122 are shown as being connected to a hardware interface 128 of a computer system 126. The computer system further comprises a processor 130 that is in communication with the hardware system 128, a memory 134, and a user interface 132. The memory 134 may be any combination of memory which is accessible to the processor 130. This may include such things as main memory, cached memory, and also non-volatile memory such as flash RAM, hard drives, or other storage devices. In some examples the memory 134 may be considered to be a non-transitory computer-readable medium.

The memory 134 is shown as containing machine-executable instructions 140. The machine-executable instructions 140 enable the processor 130 to control the operation and function of the magnetic resonance imaging system 100. The machine-executable instructions 140 may also enable the processor 130 to perform various data analysis and calculation functions.

The memory is further shown as containing a set of examination commands 142. The set of examination commands 142 contains pulse sequence instructions 144, 146 and 148. These three pulse sequence instructions 144, 146, 148 represents pulse sequence instructions for acquiring magnetic resonance data according to different magnetic resonance imaging protocols. For example, when the subject 118 is placed into the bore 106 of the magnet 104 typically different magnetic resonance imaging protocols are performed. This is represented by the set of examination commands 142. The pulse sequence instructions 144, 146, 148 are commands or data which may be converted into such commands which are used to control the magnetic resonance imaging system 100 to acquire magnetic resonance data. The memory 134 is further shown as containing magnetic resonance data 150 that was acquired according to the pulse sequence instructions 144.

The memory 134 is further shown as containing magnetic resonance data 152 that was acquired using the pulse sequence instructions 146. In this example the pulse sequence instructions 148 have not yet been executed. The memory 134 is further shown as containing an examination status 154. The examination status 154 is information about the current status of the execution of the set of examination commands 142. For example, in this illustration the examination status 154 may indicate that the protocols corresponding to the pulse sequence instructions 144 and 146 have already been executed. The examination status 154 may also contain other information about for example how long the subject 118 has been within the bore of the magnet 106. The memory 134 is further shown as containing subject data 156 that was acquired from the sensor system 122. This subject data 156 may contain data about the movement or status of the subject 118. Information such as the heart rate, skin resistance and/or breathing rate may also be included.

The memory 134 is further shown as containing a machine learning algorithm 158. The machine learning algorithm 158 takes the subject data 156 and the examination status 154 as input and outputs at least one scan decision probability 160. The at least one scan decision probability 160 could for example be an estimate that the execution of the pulse sequence instructions 148 will not be successful and will result in an abort. Another scan decision probability 160 could be the probability that the subject 118 moves too much and magnetic resonance images resulting from the acquired magnetic resonance data will be blurred or not useful.

The memory 134 is further shown as containing one or more predetermined probability ranges 162. There may for example be a predetermined probability range for each one of the at least one scan decision probability 160. The one or more predetermined probability ranges 162 are used to determine if an action should be taken on the resulting probabilities that are calculated. For example, if the at least one scan decision probability has a value within one or more of the predetermined probability ranges 162 a user prompt 164 can be displayed on the user interface 132. In this example the user prompt 164 displays a warning message 166 and a number of buttons which can select different actions. There are three different possible actions, a, b, and c labeled 168 and also a button to ignore 170 the warning and continue. The possible actions a, b and c 168 could for example be alternative pulse sequence protocols to select instead of executing the pulse sequence instructions 148 or they could be actions to modify the pulse sequence instructions 148 for example to change the resolution or otherwise modify so that the acquisition of magnetic resonance data is either accelerated or decelerated.

The computer memory 134 is further shown as containing modification instructions 172 that were received from the graphical user interface 164. The modification instructions 172 may be used to modify the pulse sequence instructions 148 or take an alternative action. After receiving the modification instructions 172 execution of the pulse sequence instructions continues and causes the set of examination commands 142 to proceed in a modified state.

The memory 134 is further shown as containing an optional historical database 174. The optional historical database 174 may for example be used to train or otherwise configure the machine learning algorithm 158. For example, if the machine learning algorithm 158 is a statistical learning algorithm then the historical database 174 may be used directly for inferring the one or more predetermined probability ranges 162. In other examples if the machine learning algorithm 158 is for example a convolution neural network then the historical database 174 maybe used for training the machine learning algorithm 158. After a completion of the set of examination commands 142 there maybe a log entry 176 that is generated.

The machine-executable instructions may then also optionally perform a feature extraction 178 on the log entry 176. This feature extraction 178 may for example be added to the optional historical database 174. The historical database 174 can then be used for later training or re-training the machine learning algorithm 158. In this way, as the magnetic resonance imaging system 100 is continually used the functioning of the machine learning algorithm 158 is improved.

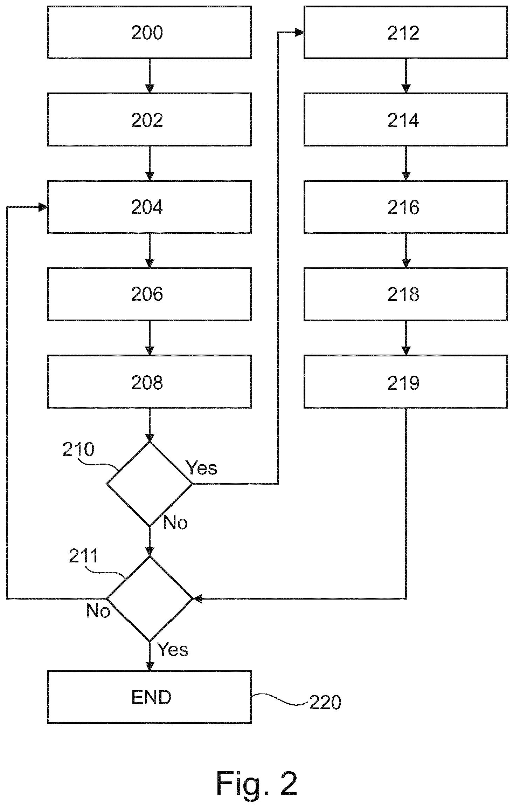

Fig. 2 shows a flowchart which illustrates a method of operating the magnetic resonance imaging system 100 of Fig. 1. First in step 200 the set of examination commands 142 are received. The set of examination commands 142 comprise the pulse sequence instructions 144, 146, 148. Next in step 202 execution of the set of examination commands 142 is initiated. The method illustrated in Fig. 2 then enters a loop. Next in step 204 the subject data 156 is received from the sensor system 122. Next in step 206 the examination status 154 is determined from the set of examination commands 142. This for example could be a listing of the operations which have been performed and the operations which are still to be performed. Next in step 208 the machine learning algorithm 158 is used to calculate the at least one scan decision probability 160 using the examination status 154 and the subject data 156 as inputs. The method then proceeds to decision box 210.

In box 210 the question is: "Is the scan decision probability within one or more of the predetermined probability ranges 162?" If the answer is "no" then the method proceeds to step 211. Step 211 is another decision box with the question: "Have all magnetic resonance imaging protocols been completed?" If the answer is "yes" the method proceeds to step 220 and the method of Fig. 2 ends. If the answer is "no" then the method proceeds back to step 204. Returning to decision box 210, if the answer is "yes" then the method then proceeds back to step 212. In step 212 execution of the set of examination commands is paused if the at least one scan decision probability is within one or more of the predetermined probability ranges.

Next, in step 214, the user prompt 164 is displayed on the user interface 132 if the at least one scan decision probability is within the one or more predetermined probability ranges. Next in step 216 the processor receives the modification instructions 172 from the user interface if the user prompt 164 is displayed. Next in step 218 the set of examination commands are modified if the modification instructions 172 are received. Next the method proceeds to step 219. In this step the execution of the set of examination commands are resumed. The method then proceeds to decision box 211 which was previously mentioned.

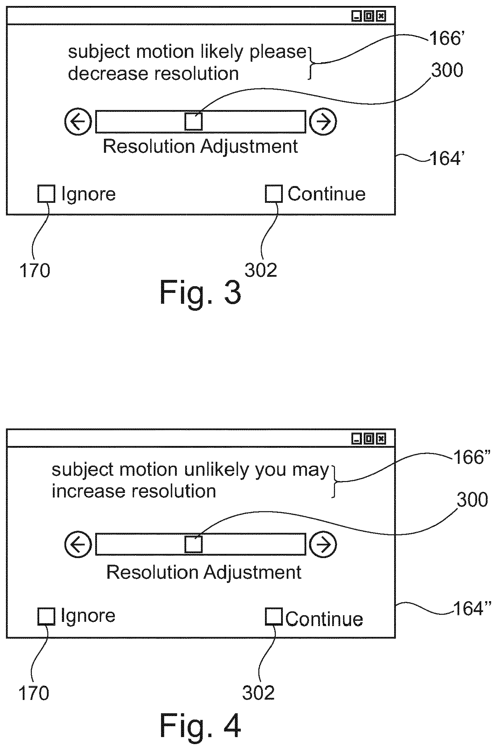

Fig. 3 illustrates an alternative example of the user prompt 164'. In the example in Fig. 3 the user prompt 164' has been displayed because the machine learning algorithm has predicted that the subject will likely move. In order to avoid the failure or abort of a magnetic resonance imaging scan it is suggested that the resolution be decreased so that the magnetic resonance data can be acquired and completed more rapidly. There is a warning message 166' that is displayed. This states that subject motion is likely, please decrease resolution. There is a slider 300 which enables the operator to adjust the resolution of the upcoming magnetic resonance imaging scan. A button 170 enables the operator to ignore this message and simply continue. There is an additional button 302 which enables the operator to continue after the slider 300 has been adjusted. In place of a slider 300 there may also be a place to numerically enter the scan resolution.

Fig. 4 shows a further example of a user prompt 164". In this example the machine learning algorithm 158 provides a prediction that the subject will move a very little and that it is possible to increase the resolution to obtain better magnetic resonance images. The message 166" is displayed and states that the subject motion is unlikely you may increase resolution. There is again the adjust resolution slider 300. The button 170 is also present and enables the operator to ignore the message 166". The continue button 302 is also present. The continue button 302 enables the operator to continue after adjusting the slider 300.

Repeated and aborted scans represent a major issue in MR imaging. It has been shown that significant extra costs are associated with motion-affected scans that have to be repeated, because of the extended examination duration.

In many cases, a trade-off between examination duration and image quality has to be made by the operating staff. When a patient is unable to stay still during an MR scan, the operator may choose a less motion-sensitive scan protocol (possibly with lower diagnostic value), or may even be forced to decide to cancel the examination. On the other hand, when a patient is very calm and time allows, a longer-than-usual scan protocol may be chosen to improve the diagnostic value of the images.

In a retrospective study, it has already been shown that there is a connection between the probability for scan repetitions (derived from MR logfiles) and the time the patient spent on the examination table. It can be assumed that including additional information about the patient's condition will increase the accuracy in predicting the repetition probability.

The probability of aborting or repeating an MR scan is difficult to estimate in advance. This is why the decision about changing a scan protocol or changing the order of scans can usually only be made after one or several scans failed because of strong motion artefacts.

Examples may overcome this problem by providing a better estimate about the probability that the next scan needs to be repeated in real-time during an ongoing examination. It further supports the operator in determining appropriate actions to optimize both the imaging workflow and the diagnostic value of the images.

Examples may comprise one or more of the following features:
(1) Correlation of real-time sensor data (i.e. patient's condition) with machine status (current state of examination) to be stored in a database;
(2) Retrospective correlation of the information from (1) with logfile information about aborted or repeated scans, to be stored in the same database;
(3) An algorithm to compare real-time sensor data and examination status with prior knowledge from the database to estimate the probability of a scan abort/repetition for the next scan;
(4) An algorithm and a display device to propose an action to the operator, based on the probability determined in (3).

Examples may be based on the combination of different data sources, describing both the examination status and the patient's (subject's) condition. Machine- and process-based data sources (one or more of the following features may be included in examples):
1. State of the examination: Can be extracted from the exam card loaded on the scanner host. The state of the examination includes the scans and actions that have already be performed, the time elapsed since the beginning of the examination, and the type of the next scan protocol
2. Modality log files: Information about stopped, aborted, or repeated scans are found here
Data sources for measuring the patient's condition (one or several from this list maybe included in examples):
1. Optical pulse sensor at finger or ECG to monitor the heart rate
2. Respirational belt to monitor the breathing rate
3. Camera to monitor the heart rate via the Vital Signs technique
4. Camera to monitor the breathing rate
5. Camera to monitor body motion
6. Electrodes to monitor the skin resistance (can be combined with the pulse sensor in a single device)
7. Microphone to monitor the amount of communication with the patient
8. One or several accelerometers attached to the patient to monitor patient body motion
9. Pressure-sensing mat placed underneath the patient to monitor patient body motion
10. The MRI data itself (e.g. k-space raw data)

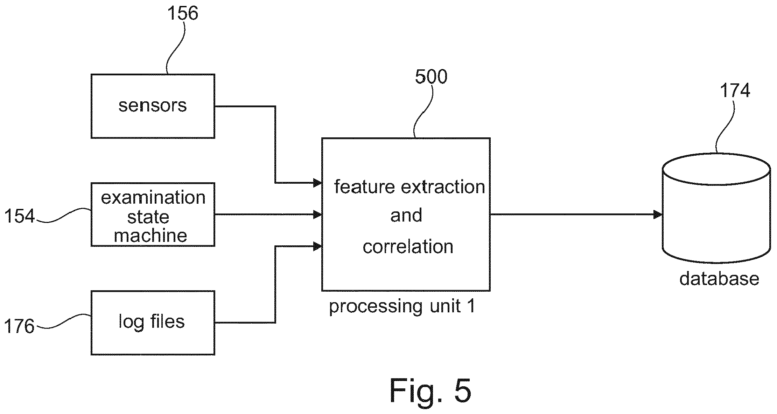

Fig. 5 illustrates an example of how to build the historical database 174. The subject data 156 acquired from sensors, the examination status 154 which is also described as being the examination state machine and the log files 176 are combined by a first processing unit that performs feature extraction and correlation of the data in 156, 154 and 176. This is then stored in historical database 174.

Fig. 5 shows how to collect the training information for the prediction algorithm. Sensor information (subject data) 156 (e.g., heart rate or skin resistance) is collected in real-time and transferred to the processing unit 1 500. At the same time, information about the current status of the examination (e.g., which scans have been performed, which scans are planned) may be derived from a state machine running on the scanner host. After the examination, these data are complemented with information from the modality log files 176 stating which of the scans have been repeated or aborted. Features and labels are extracted from the combination of data from all three sources and are stored in a database. The feature and label data is used to train a machine learning algorithm that predicts the probability of a scan having to be aborted or repeated, based on the preceding feature data of the respective examination.

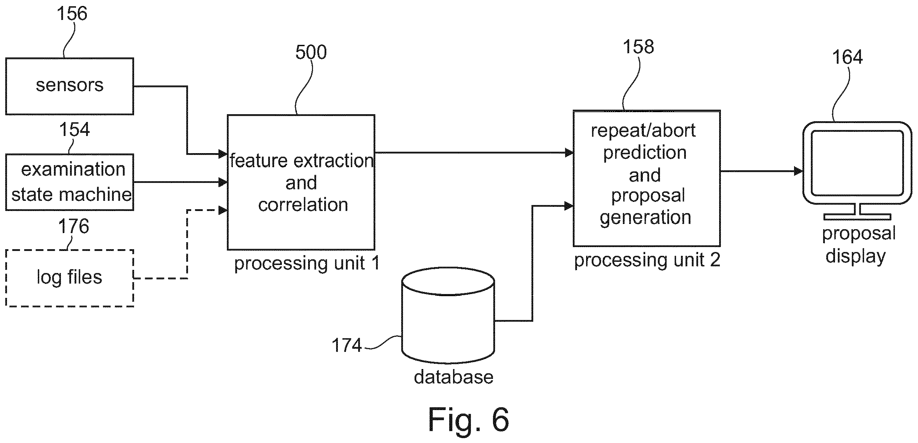

Fig. 6 illustrates one possible use of the historical database 174 and the machine learning algorithm 158. In this example the subject data 156 and the examination status 154 are combined to a feature extraction and correlation unit 500 such as is illustrated in Fig. 5. Optionally log files 176 may also be included. The output of the processing unit 1 is then input to the processing unit 2 which is equivalent to the machine learning algorithm 158. The machine learning algorithm 158 in some examples may take the historical database 174 as an input, for example if the machine learning algorithm 158 is a statistical learning method. The output of the machine learning algorithm 158 is then used to decide if a user prompt 164 should be displayed.

Fig. 6 shows the real-time prediction method. Just as in the data collection phase shown in Fig. 5, the feature extraction unit collects real-time information from sensors and scanner state machine. It may also read log files 176 in real time to know about scans that have already been stopped or earlier during the ongoing examination. A second processing unit 158 comprises a machine learning algorithm 158 that has been trained with the historical data from the database 174 (as shown in Fig. 5). Based on the real-time feature information the algorithm predicts the probability for the next scan to be aborted or repeated. In addition to an overall probability, the algorithm can also determine which features contributed most to a high probability of aborting/repeating a scan. Using a pre-defined schema, processing unit 2 will also select one or several actions based on the probability and the contributing features and proposes them to the operator using a modal dialog and/or displays them on a display device.

Possible actions that may be proposed in a user prompt may include:
1. Proposal to terminate the examination early
2. Proposal to change the order of planned scan sequences
3. Proposal to replace a scan with a less motion-sensitive protocol
4. Proposal to replace a scan with a more motion-sensitive protocol for better diagnostic value
5. Proposal to skip a scan
6. Proposal to wait for the patient to relax
7. Proposal to talk to the patient before starting the scan

In one example, the machine- and sensor-based data is complemented with relevant patient-related data from the hospital network, such as age, weight, health issues, clinical question, etc.

In one example, the information about scans that have been repeated or aborted is not derived from log files but is monitored directly by a state machine running on the scanner host.

In one example, processing unit 1 and processing unit 2 are combined in a single processing unit.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### LIST OF REFERENCE NUMERALS

- 100: magnetic resonance imaging system
- 104: magnet
- 106: bore of magnet
- 108: imaging zone
- 109: region of interest
- 110: magnetic field gradient coils
- 112: magnetic field gradient coil power supply
- 114: radio-frequency coil
- 116: transceiver
- 118: subject
- 120: subject support
- 122: sensor system
- 126: computer system
- 128: hardware interface
- 130: processor
- 132: user interface
- 134: computer memory
- 140: machine executable instructions
- 142: set of examination commands
- 144: pulse sequence instructions
- 146: pulse sequence instructions
- 148: pulse sequence instructions
- 150: magnetic resonance data
- 152: magnetic resonance data
- 154: examination status
- 156: subject data
- 158: macine learning algroithm
- 160: at least one scan decition probability
- 162: one or more predetermined probability ranges
- 164: user prompt
- 164': user prompt
- 164": user prompt
- 166: message
- 166': message
- 166": message
- 168: choice of alternative actions
- 170: ignore message
- 172: modification instructions
- 174: historical database
- 176: log entry
- 178: feature extraction
- 200: receive a set of examination commands
- 202: initiate execution of the set of examination commands
- 204: receive the subject data from the sensor system
- 206: determine an examination status using the set of examination commands
- 208: calculate at least one scan decision probability using a machine learning algorithm
- 210: Is the scan decision probability within one or more of the predetermined probability ranges?
- 211: Have all magnetic resonance imaging protocols been completed?
- 212: pause execution of the set of examination commands if the at least one scan decision probability is within one or more predetermined probability ranges
- 214: display a user prompt on the user interface if the at least one scan decision probability is within the one or more predetermined probability ranges
- 216: receive modification instructions from the user interface if the user prompt is displayed
- 218: modify the set of examination commands if the modification instructions are received
- 219: resume execution of the set of examination commands if the modification instructions have been modified using the modification instructions
- 220: end
- 300: resolution adjustment
- 302: continue button
- 500: processing unit 1

## Claims

1. A magnetic resonance imaging system (100) configured for imaging a subject (118), comprising:
- a sensor system (122) for measuring subject data (156) descriptive of a subject condition;
- a user interface (132) configured for displaying messages (166, 166', 166") and receiving modification instructions (172);
- a memory (134) for storing machine executable instructions (140); and
- a processor (130) for controlling the magnetic resonance imaging system, wherein execution of the machine executable instructions causes the processor to:
- receive (200) a set of examination commands (142), wherein the set of examination commands comprises pulse sequence instructions (144, 146, 148) for controlling the magnetic resonance imaging system to image the subject according to one or more magnetic resonance imaging protocols;
- initiate (202) execution of the set of examination commands;
wherein execution of the machine executable instructions further causes the processor to repeatedly perform the following before executing the pulse sequence instructions of each of the one or more magnetic resonance imaging protocols:
- receive (204) the subject data from the sensor system;
- determine (206) an examination status (154) using the set of examination commands;
- calculate (208) at least one scan decision probability (160) using a machine learning algorithm (158), wherein input to the machine learning algorithm comprises the subject data and the examination status, wherein the at least one scan decision probability is output of the machine learning algorithm;
- pause (212) execution of the set of examination commands if the at least one scan decision probability is within one or more predetermined probability ranges (162);
- display (214) a user prompt (164, 164', 164") on the user interface if the at least one scan decision probability is within the one or more predetermined probability ranges;
- receive (216) the modification instructions from the user interface if the user prompt is displayed;
- modify (218) the set of examination commands if the modification instructions are received; and
- resume (219) execution of the set of examination commands if the modification instructions have been modified using the modification instructions.

2. The magnetic resonance imaging system of claim, wherein the magnetic resonance imaging system further comprises a historical database (174) comprising entries, wherein each of the entries is descriptive of a prior set of examination commands stored correlated with historical subject data, and wherein each of the entries further comprises an scan status indicating a success, an abort, or repeat of the prior set of examination commands.

3. The magnetic resonance imaging system of claim 2, wherein execution of the machine executable instructions further causes the processor to train the machine learning algorithm using the historical database; and wherein the machine learning algorithm is any one of the following: a statistical classification algorithm, a neural network, a pattern recognition algorithm, a deep learning algorithm, a clustering algorithm, a k Nearest Neighbors algorithm, and a Bayesian network.

4. The magnetic resonance imaging system of any one of claims 2 or 3, wherein execution of the machine executable instructions further causes the processor to:
- generate a log entry (176) for the set of examination commands, the user data, and the scan status of the set of examination commands after execution of the set of examination commands is finished;
- perform a feature extraction (178) on the log entry; and
- append the feature extraction on the log entry to the historical database as an additional entry.

5. The magnetic resonance imaging system of any one of the preceding claims, wherein the user prompt displays a decrease resolution message (166') if the at least one scan decision probability is within a first range, and wherein the modification instructions cause the set of examination commands to decrease an imaging resolution of the pulse sequence instructions.

6. The magnetic resonance imaging system of any one of the preceding claims, wherein the user prompt displays an increase resolution message (166") if the at least one scan decision probability is below a predetermined lower value, and wherein the modification instructions cause the set of examination commands to increase the imaging resolution of the pulse sequence instructions.

7. The magnetic resonance imaging system of claim 5 or 6, wherein the user prompt comprises a selection (168) of an alternative magnetic resonance imaging protocol.

8. The magnetic resonance imaging system of any one of the preceding claims, wherein the user prompt displays a choice or a suggestion to:
- terminate execution of the set of examination commands,
- change an ordering of remaining magnetic resonance imaging protocols,
- delay execution of the set of examination commands until the subject relaxes,
- skip execution of one or more of the magnetic resonance imaging protocols,
- speak with the subject, and
- combinations thereof.

9. The magnetic resonance imaging system of any one of the preceding claims, wherein the examination status comprises any one of the following:
- remaining magnetic resonance imaging protocols to be performed;
- magnetic resonance imaging protocols already performed;
- a time elapsed since initiation of the set of examination commands;
- a type of a next magnetic resonance imaging protocol to be performed; and
- combinations thereof.

10. The magnetic resonance imaging system of any one of the preceding claims, wherein the subject data further comprises:
- any subject-specific data accessible through the hospital network,
- an age of the subject,
- a weight of the subject,
- a height of the subject,
- a health status of the subject,
- a medical history of the subject,
- one or more questionnaire responses of the subject, and
- combinations thereof.

11. The magnetic resonance imaging system of any one of the preceding claims, wherein execution of the machine executable instructions further causes the processor to calculate the subject data at least partially from magnetic resonance data acquired during execution of the set of examination commands.

12. The magnetic resonance imaging system of any one of the preceding claims, wherein the sensor system comprises any one of the following:
- an optical finger pulse sensor configured for monitoring a subject heart rate;
- an ECG system configured for monitoring the subject heart rate;
- a respiratory belt configured for monitoring a subject breathing state;
- a breathing tube configured for monitoring the subject breathing state;
- a heart rate camera system configured for measuring the subject heart rate using facial color changes;
- a motion monitor camera system configured for measuring a subject position;
- a breathing monitor camera system configured for measuring the subject breathing state;
- a skin resistance system configured for measuring skin resistance data of the subject;
- a microphone system configured for quantifying a cumulative speaking duration of the subject; and
- combinations thereof.

13. A computer program product comprising machine executable instructions (140) for execution by a processor (130) controlling a magnetic resonance imaging system (100), wherein the magnetic resonance imaging system comprises a sensor system (122) for measuring subject data (156) descriptive of a subject condition, wherein the magnetic resonance imaging system further comprises a user interface (132) configured for displaying messages (166, 166', 166") and receiving modification instructions, wherein execution of the machine executable instructions causes the processor to:
- receive (200) a set of examination commands (142), wherein the set of examination commands comprises pulse sequence instructions (144, 146, 148) for controlling the magnetic resonance imaging system to image the subject according to one or more magnetic resonance imaging protocols;
- initiate (202) execution of the set of examination commands;
wherein execution of the machine executable instructions further causes the processor to repeatedly perform the following before executing the pulse sequence instructions of each of the one or more magnetic resonance imaging protocols:
- receive (204) the subject data from the sensor system;
- determine (206) an examination status (154) using the set of examination commands;
- calculate (208) at least one scan decision probability (160) using a machine learning algorithm (158), wherein input to the machine learning algorithm comprises the subject data and the examination status, wherein the at least one scan decision probability is output of the machine learning algorithm;
- pause (212) execution of the set of examination commands if the at least one scan decision probability is within one or more predetermined probability ranges (162);
- display (214) a user prompt (164, 164', 164") on the user interface if the at least one scan decision probability is within the one or more predetermined probability ranges;
- receive (216) the modification instructions from the user interface if the user prompt is displayed;
- modify (218) the set of examination commands if the modification instructions are received; and
- resume (219) execution of the set of examination commands if the modification instructions have been modified using the modification instructions.

14. A method of operating a magnetic resonance imaging system (100), wherein the magnetic resonance imaging system comprises a sensor system (122) for measuring subject data (122) descriptive of a subject condition, wherein the magnetic resonance imaging system further comprises a user interface (132) configured for displaying messages (166, 166', 166") and receiving modification instructions (172), wherein the method comprises:
- receiving (200) a set of examination commands (142), wherein the set of examination commands comprises pulse sequence instructions (144, 146, 148) for controlling the magnetic resonance imaging system to image the subject according to one or more magnetic resonance imaging protocols;
- initiating (202) execution of the set of examination commands;
wherein the method comprises repeatedly perform the following before executing the pulse sequence instructions of each of the one or more magnetic resonance imaging protocols:
- receiving (204) the subject data from the sensor system;
- determining (206) an examination status (154) using the set of examination commands;
- calculating (218) at least one scan decision probability (160) using a machine learning algorithm (158), wherein input to the machine learning algorithm comprises the subject data and the examination status, wherein the at least one scan decision probability is output of the machine learning algorithm;
- pausing (212) execution of the set of examination commands if the at least one scan decision probability is within one or more predetermined probability ranges (162);
- displaying (214) a user prompt (164, 164', 164") on the user interface if the at least one scan decision prbability is within the one or more predetermined probability ranges;
- receiving (216) the modification instructions from the user interface if the user prompt is displayed;
- modifying (218) the set of examination commands if the modification instructions are received; and
- resuming (219) execution of the set of examination commands if the modification instructions have been modified using the modification instructions.
